# EUROPÄISCHE PATENTSCHRIFT

(11) **EP 2 142 136 B1**
(45) Veröffentlichungstag und Bekanntmachung des Hinweises auf die Patenterteilung: **25.01.2017**
(21) Anmeldenummer: 08757938.9
(22) Anmeldetag: 15.04.2008
(51) Int. Cl.: A61C 8/00

(54) **ZAHNIMPLANTATSYSTEM**
DENTAL IMPLANT SYSTEM
SYSTÈME D'IMPLANT DENTAIRE

(30) Priorität: 17.04.2007 DE 102007018453; 02.04.2008 DE 102008017086; 02.04.2008 DE 102008017085
(43) Veröffentlichungstag der Anmeldung: 13.01.2010
(73) Patentinhaber: Indi Implant Systems GmbH, 02826 Görlitz (DE)
(72) Erfinder: MENZEL, Michael, 02827 Görlitz (DE); RÖRUP, Henning, 21385 Amelinghausen (DE); ARAPOGLOU, Konstantinos, 76228 Karlsruhe (DE)
(74) Vertreter: Kaufmann, Sigfrid
(86) Internationale Anmeldenummer: PCT/DE2008/000627
(87) Internationale Veröffentlichungsnummer: WO 2008/125097

(56) Entgegenhaltungen:
- EP-A- 1 013 236
- WO-A-01/34056
- WO-A-2005/065571
- WO-A-2006/084346
- DE-A1- 19 513 881
- DE-A1-102004 055 831
- GB-A- 1 431 563
- RU-C1- 2 146 113
- US-A- 5 759 036
- US-A1- 2004 185 419
- US-A1- 2006 252 009
- US-A1- 2006 292 523

## Beschreibung

Die Erfindung betrifft ein aus einem Implantat und einer Suprastruktur bestehendes Zahnimplantatsystem.

In Deutschland wird jährlich rund 700000 mal künstlicher Zahnersatz implantiert. Üblicherweise besteht dieser aus drei Komponenten: einer künstlichen Wurzel (Wurzelimplantat), einem Verbindungsstück, dem so genannten Abutment, und einer Suprakonstruktion wie die z. B. einer Krone oder einer Brücke.

Zum Einsetzen des künstlichen Zahnersatzes müssten zuerst die Reste des defekten Zahnes, wie z. B. dessen Wurzeln, vollständig entfernt werden. Anschließend wird in den Kiefer eine Bohrung eingebracht bzw. in diesen ein Gewinde geschnitten und das Wurzelimplantat eingeschlagen bzw. eingeschraubt. Das Implantat wird deshalb meistens in Form eines Zylinders bzw. eines Konus hergestellt. Nach dem Einsetzen in den Kiefer muss das Implantat drei bis sechs Monate lang einheilen, d. h. fest mit dem Kiefer verwachsenen. In dieser Zeit darf das Implantat mechanisch nicht belastet werden. Nach dem Einheilen wird üblicherweise das Abutment mit dem Implantat verschraubt oder verklebt und anschließend die Suprakonstruktion auf das Abutment aufgebracht. Das Abutment trägt einen sog. Aufbaustift, welcher zur mechanischen Befestigung der Suprakonstruktion am Abutment dient.

Dieser Aufbau bringt jedoch mehrere Nachteile mit sich. Konstruktionsbedingt entsteht zwischen dem Implantat und dem Abutment ein bakteriologisch besiedelbarer Spalt. Zahnfleischtaschen entlang dem Abutment in Richtung Knochen bilden offene Eintrittsstellen für Bakterien. Die von den Bakterien als Ausscheidungsprodukte gebildeten Säuren verhindern ein Verwachsen des Zahnfleisches mit dem Implantat/Abutment im Bereich des Spalts. Der Kieferknochen bildet sich immer entsprechend der biologischen Breite zurück; oft treten zusätzlich noch Entzündungen des Zahnfleisches und weitere Knocheneinbrüche sowie folgend weiterer Zahnfleischverlust auf. Als Konsequenz davon entstehen bakterielle Taschen, die, da sie dem Patienten selbst nicht zugänglich sind, teure und für den Patienten lästige zahnärztliche Recallmaßnahmen zur bakteriellen Taschensäuberung erforderlich machen und regelmäßig auch zum Verlust des Implantates führen. Die Durchführung der Taschensäuberung hat außerdem den Nachteil, dass das Zahnfleisch, bedingt durch diese, im Bereich oberhalb des Spalts zwischen Abutment und Implantat nicht mit dem Abutment verwachsenen kann. Hinzu kommt, dass die zwischenzeitlich unvermeidbare schlechte Zugänglichkeit der Zahnfleischtaschen eine ordentliche Mundhygiene in diesem Bereich verhindert und das Implantat infolgedessen vom Patienten als sehr unangenehm empfunden wird. Während dieser Zeit schreitet die Entzündung und Zerstörung des gesunden Gewebes weiter voran.

Ein weiterer Nachteil ist, dass die Schraubverbindungen zwischen Wurzelimplantat und Abutment hohen mechanischen Belastungen ausgesetzt sind. Dadurch kommt es regelmäßig zu Mikrobewegungen zwischen dem Implantat und dem Abutment und nicht zuletzt zu Schraubenbrüchen mit der Folge hoher Kosten. Wird die Schraubenverbindung mit Zement oder Kleber fixiert, so kann außerdem austretendes Material zu Reizungen des Zahnfleisches führen.

Die Wurzelimplantate sind üblicherweise als in verschiedenen Größen abgestufte, vorgefertigte, rotationssymmetrische Standardimplantate ausgeführt. Aufgrund der CE-Vorschriften für die Sterilisierung medizinischer Gegenstände darf der die Implantierung durchführende Zahnarzt während dieser die vorgefertigten Implantate nicht individuell anpassen. Aufgrund der vorgegebenen Abstufung müssen deshalb oft Kompromisse bezüglich der Größe der eingesetzten Implantate eingegangen werden. Nachteilig ist auch, dass das Implantat nicht an die durch das Einbohren des Implantatbettes in den kammförmigen Kiefer entstehenden Knochendehiszenzen angepasst werden kann. Infolgedessen weisen solche Implantate eine wesentlich geringere Knochenkontaktfläche und eine damit verbundene schlechtere Verankerung auf, als dies bei individueller Anpassung möglich wäre. Darüber hinaus werden die anatomischen Weichteilansprüche des Zahnfleisches nicht berücksichtigt. Ästhetik und Hygiene werden damit stark eingeschränkt. Die nicht kompensierten Knochendehiszenzen sind regelmäßig ursächlich für die Bildung von unerwünschten Zahnfleischtaschen. Schließlich entstehen hohe Lagerhaltungskosten, da die vorgefertigten Standardimplantate in vielen Größen und Formen vorgehalten werden müssen.

Die Befestigung der Suprakonstruktion am Abutment mit Hilfe eines Aufbaustifts führt zu ungünstigen Kräfteverteilungen und Hebelverhältnissen, wodurch eine erhöhte Bruchgefahr besteht. Bei verschraubten Aufbaustiften treten außerdem noch nachweisbar zerstörerische Mikrobewegungen auf.

In den letzten Jahren wurde ein erheblicher Fortschritt in der Implantattechnik erreicht; zahlreiche Entwicklungen richten sich auf die Beseitigung der o. g. Probleme.

So wird in DE 196 47 489 A1 vorgeschlagen, auf das Abutment an der Kontaktstelle zum enossalen Implantatkörper eine direkt aufgalvanisierte, plastisch verformbare Metallschicht aufzubringen. Damit soll die Bildung des bakteriologisch besiedelbaren Spaltes zwischen Abutment und Implantat vermieden werden. In analoger Weise wird in DE 196 47 490A1 angeregt, zwischen dem Implantat und dem Implantataufbau eine Goldscheibe einzubringen. Beide Lösungen führen zwar zu einer Minimierung des Spalts, es steht jedoch zu erwarten, dass der Kontakt zwischen Titan, Gold und Zahnfleisch sowie Speichel irritative chemische Reaktionen hervorruft. Eine bakteriologische Besiedlung ist zudem nicht auszuschließen.

In WO2004/058096 A1 wird ein Abutment beschrieben, das mit einer axialen durchgängigen Bohrung versehen ist. Es ist vorgesehen, das Abutment mit dem Implantat zu verschrauben, wobei die Verschraubung zusätzlich mit Zement fixiert wird. Die durchgängige Bohrung dient dazu, dass während des Verklebens überschüssiger Zement durch diese herausgedrückt wird und sich auf dem flachen Kopf des Implantats sammelt. Von dem Kopf kann der Zement einfach entfernt werden. Damit können Zahnfleischentzündungen durch an anderen Stellen austretenden Zement verhindert werden.

DE 10 2005 027 402 A1 offenbart ein Verfahren zur Herstellung eines individualisierten Ersatzzahns, der über einen Verbindungszapfen in einem eingeheilten Implantat aufgenommen werden kann. Um den Aufwand bei der Erstellung von Zahnersatz zu verringern, wird vorgeschlagen, den Ersatzzahn einteilig herzustellen. Damit kann auf ein separates Abutment verzichtet werden. Hierdurch wird unbestritten eine Vereinfachung erzielt, das Problem des bakteriologisch besiedelbaren Spalts bleibt jedoch bestehen.

In EP 0 967 931 B1 wird ein Zahnimplantat vorgestellt, das aus einem konischen Schaftabschnitt und einem divergenten Kopfabschnitt besteht, wobei beide Abschnitte integral ausgebildet bzw. als ein verbundenes Teil ausgeführt sind. Das Zahnimplantatsystem umfasst außerdem ein zum Zahnimplantat passendes Abutment, das zum Stützen eines Zahnersatzes/einer Krone dient. Das Abutment wird mit Hilfe einer Schraube in den konischen Schaftabschnitt des Zahnimplantats eingeschraubt.

Durch die einteilige bzw. integrale Ausführung von Schaft- und Kopfabschnitt wird zwar ein Mikrospalt im enossalen Bereich vermieden, die Ausbildung eines bakteriologisch besiedelbaren Spaltes zwischen Schaft-/Kopfabschnitt und Abutment im gingivalen Bereich ist jedoch unvermeidlich.

In EP 0 891 163 B1 wird ein dentales Stützlager beschrieben, das aus einem Implantat und einem Abutment aufgebaut ist. Die Oberkante des Abutments und entsprechend die Unterkante der daran befestigten Krone sind beim Austritt aus dem Zahnfleisch an das entsprechende Austrittsprofil und die Größen des zu ersetzenden Zahnes angepasst.

Dadurch, dass die Grenze zwischen Abutment und Krone an den Verlauf des Zahnfleisches angepasst wird, werden ästhetische und funktionelle Vorteile gegenüber rotationssymmetrischen Lösungen erreicht. Die Grenze zwischen Abutment und Krone liegt jedoch genau auf der Höhe des Zahnfleischsaums oder darüber. Da sich das Zahnfleisch im Laufe der Zeit üblicherweise zurückbildet, lässt sich nicht vermeiden, dass nach einiger Zeit das metallische Abutment über den Zahnfleischsaum reicht und sichtbar wird.

Alternativ zu dem vorstehend beschriebenen Zahnersatz ist es allgemeiner Stand der Technik, ein rotationssymmetrisches Implantat zu verwenden, bei dem die enossalen Teile des Implantats, das Abutment und ggf. der Aufbaustift aus einem Stück gefertigt oder zumindest nahtlos gefügt sind (s. DE 10 2005 001 185 A1).

Derartige Implantate vermeiden demgemäß von vornherein einen Spalt im Grenzbereich zwischen Kieferknochen und Zahnfleisch, das Zahnfleisch kann mit dem Implantat problemlos verwachsen, da Mikrobewegungen vermieden werden. Trotzdem konnten sich diese Implantate aus mehreren wesentlichen Gründen nicht durchsetzen. Ein Grund ist, dass der Aufbaustift konstruktionsbedingt in den Mundraum ragt. Dies führt unweigerlich zu mechanischen Belastungen des Implantats während des Einwachsens und daraus folgend häufig zum Fehlschlagen der Implantierung. Ein weiterer Grund ist, dass aufgrund der Rotationssymmetrie des Implantats dieses am Übergang von Krone zum Zahnfleisch sichtbar bleibt, was insbesondere bei der Verwendung von Titan aus ästhetischen Gründen nicht akzeptabel ist. Besonders nachteilig ist, dass die durch die Bohrung in den kammförmigen Kieferknochen verursachten vestibulären/buccalen und palatinalen/lingualen Knochendehiszenzen von der Form des Implantates nicht berücksichtigt werden.

Weitere Zahnimplantate bzw. Zahnimplantatsysteme mit rotationssymmetrischem Querschnitt des unteren enossalen Bereichs und/oder Gewinde zum Einschrauben in den Knochen sind aus WO 2005/065571 A, US 2006/252009 A1, DE 10 2004 055 831 A1, US 5 759 036 A, WO 2006/084346 A, EP 1 013 236 A und RU 2 146 113 C1 bekannt.

Auch beschreibt US 2004/0185419 A1 ein einstückiges Implantat mit einem Gewinde im enossalen Bereich.

Ein einstückiges Implantat aus Zirkonoxid, dessen transgingivaler Bereich erst im implantierten Zustand auf die benötigte Form geschliffen wird, ist in WO 01/34056 A1 offenbart.

Aus GB-A-1 431 563 ist ein Implantat aus einer Kohlenstoffmodifikation bekannt, dessen Oberfläche durch zirkular umlaufende Rillen, die im Längsschnitt eine sägezahnartige Struktur aufweisen, vergrößert ist.

DE 195 13 881 A1 beschreibt ein Verfahren zur Herstellung eines Implantats, bei dem ein Rohling entsprechend der natürlichen Kavität des Zahnwurzelbereichs durch Kopierfräsen nachgebildet wird.

US2006/0292523 A1 offenbart ein Zahnimplantatsystem mit einem Implantat und einer darauf aufgebauten Suprakonstruktion. Das Implantat weist einen mit Rillen strukturierten unteren enossalen Bereich, einen sich nach oben hin aufweitenden oberen enossalen Bereich mit aufgerauter Oberfläche, einen transgingivalen Bereich mit glatter Wandfläche und einen transgingivalen hexagonalen Implantatkopf auf. Die Grenze zwischen dem aufgerauten oberen enossalen Bereich und dem glatten transgingivalen Bereich ist krumm und die koronale Kante des transgingivalen Bereichs ist auch krumm. In einem Querschnitt senkrecht zur Implantatsachse hat der transgingivale Bereich eine nichtrotationssymmetrische Form.

Aufgabe der Erfindung ist es, die Nachteile des Standes der Technik zu beseitigen. Insbesondere soll ein ästhetisch ansprechendes, am Zahnfleischsaum unsichtbares Zahnimplantatsystem geschaffen werden, das ein gutes Verwachsen des Zahnfleisches im gingivalen Bereich ermöglicht und eine mechanisch stabile und langlebige Verbindung eines Zahnaufbaus mit dem Kieferknochen gewährleistet.

Diese Aufgabe wird erfindungsgemäß durch die kennzeichnenden Merkmale des Anspruchs 1 gelöst; weitere vorteilhafte Ausführungen ergeben sich aus den Ansprüchen 2 bis 10.

Ausgegangen wird von einem Zahnimplantatsystem, das aus einem Implantat und einer darauf aufgebauten Suprakonstruktion besteht. Das Implantat setzt sich aus einem wabenförmig strukturierten unteren enossalen Bereich, einem sich nach oben hin aufweitenden oberen enossalen Bereich mit aufgerauter Oberfläche, einem transgingivalen Bereich mit glatter Wandfläche und einem transgingivalen Implantatkopf zusammen. Alle diese Bereiche schließen naht- und spaltlos aneinander an.

Der untere enossale Bereich ist üblicherweise rotationssymmetrisch zylindrisch/konisch, kombiniert rotationssymmetrisch zylindrisch/konisch, rotationssymmetrisch stufenförmig zylindrische/konisch oder kombiniert rotationssymmetrisch stufenförmig zylindrisch/konisch geformt. Bevorzugt ist er mit einer Wabenstruktur mit Spitzen an den Eckpunkten versehen, die für ein gutes Einwachsverhalten in den Kieferknochen bekannt ist. Nach Maßgabe der Erfindung weist der obere enossale Bereich jeweils in vestibulärer/bukkaler Richtung und in palatinaler/lingualer Richtung eine Verkürzung auf. Entsprechend ist der obere enossale Bereich, an den Stellen, an denen er seitlich am Kieferkamm anliegt, höher ausgeführt. Dadurch wird das Implantat genau an die patientenspezifische Kammform des Kiefers angepasst und infolgedessen werden die durch die Bohrung des Implantatbettes entstehenden Knochendehiszenzen vollständig vom Implantat berücksichtigt. Dadurch dass sich der obere enossale Bereich nach obenhin aufweitet, wird bei der Implantierung eine größere Knochenkontaktfläche und folglich ein besserer Halt des Implantats im Kieferknochen erreicht.

Der glatte transgingivale Bereich ist entsprechend der Kavität im Zahnfleisch des Patienten geformt. Bedingt durch diese anatomische Ausformung weist der transgingivale Bereich bei jedem Längs- und jedem Querschnitt eine andere Form als bei dem entsprechenden davor oder danach liegenden Längs- bzw. Querschnitt auf. Der transgingivale Bereich wird nach oben durch einen individuell geformten, dreidimensional differenziert in Höhe, Breite und Tiefe gestalteten transgingivalen Implantatkopf begrenzt, d. h. der Implantatkopf bildet die "Deckelfläche" des Implantats. Er weist ebenfalls bei jedem Längsschnitt und jedem Querschnitt eine andere Form als bei dem davor oder danach liegenden Längsschnitt bzw. Querschnitt auf. Die so durch den transgingivalen Bereich und den transgingivalen Implantatkopf gebildete, umlaufende Kante entspricht hierbei der Präparationsgrenze und ist so geformt, dass diese nach erfolgter Implantierung knapp, typischerweise 1 mm, unterhalb des Zahnfleischsaums verlaufen wird.

Der transgingivale Implantatkopf weist in seiner Mitte einen flachen Bereich auf. Dieser ist umgeben von einem steiler abfallenden Teilbereich, der den flachen Teilbereich mit dem transgingivalen Bereich verbindet. Dabei ist das durch den flachen Bereich des Implantatkopfs gebildete Plateau genau so hoch angeordnet, dass es in approximaler Richtung auf Höhe der oberen Zahnfleischbegrenzung liegt.

Erfindungsgemäß, ist der steilere Teilbereich des Implantatkopfes in vestibulärer/buccaler Richtung sowie in palatinaler/lingualer Richtung länger und steiler als in den übrigen Richtungen.

Somit wird der obere enossale Bereich an die patientenspezifische Kammform des Kiefers angepasst.

Auf die gesamte Fläche des Implantatkopfes ist eine Suprakonstruktion mit ihrer Unterseite, die der Form des Implantatkopfes angepasst ist, aufgeklebt oder - zementiert. Diese Verbindung nimmt einen Großteil der zwischen Implantat und Suprakonstruktion wirkenden Kräfte auf. Zur Vermeidung einer seitlichen Verschiebung und um eine weitere mechanische Stabilisierung zu erreichen, ist zusätzlich ein Aufbaustift ungefähr im Zentrum der Präparationsfläche in den transgingivalen Bereich des Implantats eingeschraubt und auf der anderen Seite mit der Suprakonstruktion verbunden. Die Basalfläche des Aufbaustifts liegt auf dem flachen Teil des transgingivalen Implantatkopfes auf und wirkt als kraftkompensierender Kippmeider und als kraftaufnehmender und übertragender Teil.

Das Implantat ist vorteilhafterweise aus einem Stück (Rohling) hergestellt. Als medizintechnisch bewährte Materialien bieten sich hierbei Titan und Zirkonoxid sowie alle zur Zahnimplantation geeigneten Materialien an.

Das erfindungsgemäße Zahnimplantatsystem zeigt gegenüber den üblicherweise verwendeten Systemen mehrere Vorteile.

Wesentlicher Vorteil des Implantatsystems ist, dass dieses ohne Abutment auskommt, wodurch ein bakteriologisch besiedelbarer Spalt im Grenzbereich zwischen Kieferknochen und Zahnfleisch von vornherein vermieden wird. Da sich die Präparationsgrenze knapp unterhalb des Zahnfleischsaums befindet, ist diese dem Patienten zugänglich und kann von diesem bequem gereinigt werden. Die Notwendigkeit von zahnärztlichen Taschensäuberungen entfällt. Außerdem kann das Implantat schon während der Einwachsphase des enossalen Bereichs in den Kieferknochen gleichzeitig im transgingivalen Bereich mit dem Zahnfleisch verwachsen. Dies ist besonders vorteilhaft, wenn das Zahnfleisch durch das Vorgehen zur Implantation noch "frisch und blutig" ist. Da keine Notwendigkeit zur Taschensäuberung besteht, muss das an das Implantat gewachsene Zahnfleisch auch nicht später wieder gelöst werden. Dies hat zur Folge, dass teure und für den Patienten lästige Recallmaßnahmen vermieden werden und außerdem, da sich nicht wie sonst üblich, Zahnfleischtaschen bilden, das Implantat vom Patienten kaum noch als Fremdkörper empfunden wird, da die Maßnahmen zur Mundhygiene in diesem Bereich einfach und vor allem sicher zu bewältigen sind.

Durch das Wegfallen der Verschraubung zwischen Implantat und Abutment, werden die sonst konstruktiv bedingten Mikrobewegungen zwischen Implantat und Abutment, die häufig die Ursache für Schraubenbrüche aber auch für eine Rückbildung des umgebenden Kieferknochenabschnitts und folgend auch die Ursache für die Rückbildung des über dem Kieferknochen befindlichen und eben durch den Knochen unterstützten und stabilisierten Zahnfleisches sind, ausgeschlossen.

Durch die individuelle, an die Anatomie des Patienten angepasste Größe und Form des unteren enossalen Bereichs, wird eine größtmögliche Knochenkontaktfläche erreicht. Durch die sich nach oben aufweitende Form sowie die genaue Anpassung des oberen enossalen Bereichs an die Kammform des Kieferknochens und die damit verbundene vollständige Anpassung an die durch Implantatbettbohrung entstehenden Knochendehiszenzen, wird gleichermaßen eine große Auflagefläche im oberen Bereich des Kieferknochens gewährleistet. Bedingt durch seine strukturierte bzw. raue Oberfläche kann der enossale Bereich des Implantats mit dem Kieferknochen zügig und sicher verwachsen.

Die einzige Verbindungsstelle des Zahnimplantatsystems ist so gewählt, dass sie einerseits vom Patienten selbst gereinigt werden kann, andererseits jedoch mechanische Belastungen während der Einwachsphase durch mechanischen Kontakt, wie z. B. mit anderen Zähnen, weitgehend vermieden werden.

Im Gegensatz zu den bislang verwendeten Zahnimplantatsystemen, bei denen die mechanische Verbindung zwischen Implantat und Suprastruktur hauptsächlich über ein Abutment/einen Aufbaustift erfolgt, nimmt bei dem erfindungsgemäßen System eine großflächige stoffschlüssige Verbindung, gebildet durch Kleber oder Zement zwischen Implantatkopf -und Unterseite der Suprastruktur, die mechanischen Belastungen auf. Der verwendete Prothetikpfosten hat eine unterstützende Funktion, verhindert ein seitliches Verschieben während des Einbringens der Suprastruktur, und fungiert durch die basale Auflage auf dem flachen Teil des transgingivalen Implantatkopfes bei seitlich einfallenden Kräften als kraftaufnehmendes Element.

Es ist erfindungsgemäß auch vorgesehen, dass der untere und obere enossale Bereich des Zahnimplantatsystems nichtrotationssymmetrisch ausgeformt ist, wobei seine Geometrie der Überlagerung von mindestens zwei rotationssymmetrischen geometrischen Körpern entspricht, deren Symmetrieachsen zueinander parallel versetzt oder derart zueinander verkippt sind, dass sie sich zumindest im unteren enossalen Bereich schneiden. Die geometrischen Körper überlappen sich immer zumindest im oberen enossalen Bereich, sodass dort nichtrotationssymmetrische geschlossene Querschnittsflächen gebildet werden. Der untere enossale Bereich kann zahnwurzelartig ausgebildet sein oder gleichfalls geschlossene Querschnittsflächen aufweisen.

Aufgrund der nichtrotationssymmetrischen Form des enossalen Bereichs bildet das eingesetzte Implantat mit den Kieferknochen ein Widerlager, das vergleichsweise hohen Dreh- und Kippbelastungen standhält. Zudem ermöglicht diese Ausgestaltung die eindeutige Positionierung und Fixierung des Implantates im Kieferknochen.

Für die Teile des oberen und unteren enossalen Bereichs, die geschlossene Querschnittsflächen aufweisen, sind bevorzugt Geometrien gewählt, die der Überlagerung von zylindrischen geometrischen Körpern mit kegelförmigen Spitzen entsprechen. Die Zylinder können dieselben aber auch nicht dieselben Radien haben. Die Symmetrieachsen der Körper sind so angeordnet, dass sie sich zumindest im unteren enossalen Bereich schneiden. Die Kippwinkel der Symmetrieachsen zueinander betragen üblicherweise 1° bis 45°.

Für zahnwurzelartig ausgebildete Teile des unteren enossalen Bereichs sind bevorzugt Geometrien gewählt, die der Überlagerung von parallel angeordneten, konischen geometrischen Körpern entsprechen.

Das Verfahren zur Herstellung eines Zahnimplantatsystems und zu dessen Einbringung in den Kiefer bzw. Mundraum des Patienten umfasst die folgenden Schritte:
Zuerst werden die Kieferform, und die Form des Zahnfleisches des zu behandelnden Patienten mit CT (Computertomographie), DVT (Volumentomographie) oder alternativ dazu mit OPG aufgenommen.

An einem Rohling, aus dem das gesamte Implantat hergestellt werden soll, wird zuerst anhand der ermittelten anatomischen Diagnosedaten der enossale Abschnitt mithilfe der CAD/CAM-Technik geformt. Dieser Abschnitt ist rotationssymmetrisch zylindrisch/konisch, kombiniert rotationssymmetrisch zylindrischen/konisch, rotationssymmetrisch stufenförmig zylindrisch/ konisch oder kombiniert rotationssymmetrisch stufenförmig zylindrisch/konisch geformt. Um ein gutes Einwachsens in den Knochen zu ermöglichen, ist der enossale Abschnitt mit einer Struktur versehen. Vorzugsweise wird hierbei eine bewährte Wabenstruktur, die Spitzen an ihren Eckpunkten aufweist, gewählt.

Anschließend wird angrenzend an den enossalen Abschnitt entsprechend den anatomischen Diagnosedaten ein weiterer enossaler Bereich durch CAD/CAM geformt. Zur individuellen Anpassung an die Kammform des Kiefers wird hierbei in vestibulärer/bukkaler Richtung eine stärkere und in palatinaler/lingualer Richtung eine geringere Verkürzung eingearbeitet. Auf diese Weise werden später bei der Bohrung des Implantatbettes entstehende Knochendehiszenzen von der Implantatform vollständig berücksichtigt.

Danach wird ein transgingivaler Bereich anhand der ermittelten anatomischen Diagnosedaten durch manuelle Bearbeitung oder ggf. durch CAD/CAM hergestellt. Die individuelle Ausformung, führt dazu, dass in jedem Fall der transgingivale Bereich bei jedem Längsschnitt und jedem Querschnitt eine andere Form als bei dem entsprechenden davor oder danach liegenden Längsschnitt bzw. Querschnitt aufweist.

Daraufhin wird der den transgingivalen Bereich nach oben begrenzende transgingivale Implantatkopf durch manuelle Bearbeitung oder ggf. durch CAD/CAM hergestellt. Der Implantatkopf wird nach den diagnostischen Vermessungsdaten der Form sowie des Randverlaufs der Kavität in der Gingiva so ausgeformt, dass die durch die Präparationsfläche und den transgingivalen Bereich gebildete Kante nach der Implantation unterhalb des Zahnfleischsaums verläuft. Ungefähr in der Mitte des Implantatkopfes wird ein flacher Teilbereich und diesen umgebend ein steiler abfallender Teilbereich geformt. Durch den steileren Teilbereich werden der flachere Teilbereich und der transgingivale Bereich miteinander verbunden. Außerdem ist eine Gewindebohrung für die Aufnahme eines Aufnahmepfostens in den flachen Teilbereich des Implantatkopfes eingebracht.

Als nächstes wird der transgingivale Bereich und der transgingivale Implantatkopf geglättet. Das Glätten erfolgt vorzugsweise durch mechanisches manuelles Polieren bzw. durch CAD/CAM. Danach erfolgt das Aufrauen der Oberfläche des gesamten enossalen Bereiches des Implantates mittels geeigneter Techniken.

Als nächster Schritt erfolgt ein stufenweises Heranführen des Durchmessers des zu bohrenden Implantatbettes. Dazu wird als erstes in den Kieferknochen das Implantatbett stufenweise mit entsprechenden Bohrern, beginnend bei einem ca. 1 mm kleineren bis hin zu einem 0,5 mm kleineren Durchmesser als der untere enossale Bereich des Implantates, eingebohrt. Anschließend wird anhand der Bohrung oder durch weitere Testverfahren die Härte bzw. Qualität des Knochens ermittelt. Da sich die Knochenqualität und -härte während der Erweiterung eines Bohrloches drastisch ändern können, wird der Durchmesser des Bohrloches stufenweise in Schritten von 0,1 mm vergrößert. Mit jeder erfolgten Bohrung wird die Knochenqualität neu ermittelt und der Durchmesser der Bohrung mit Hilfe von geeigneten Messinstrumenten und Verfahren geprüft. Die Bohrung wird solange schrittweise vergrößert, bis die Knochenqualität und die Größe des Bohrloches optimal aufeinander abgestimmt sind.

Danach erfolgt ein Einsetzen des Implantats durch Einklopfen des enossalen Bereichs in das Bohrloch.

Anschließend heilt das Implantat über einen Zeitraum von 3 bis 6 Monaten in den Kiefer ein.

In einem weiteren Schritt wird ein geeigneter Aufbaustift gewählt bzw. hergestellt. Zeigt das Implantat keine Angulation, so kann ein gerader, vorkonfektionierter Aufbaustift, der auf einer Seite ein Gewinde zum Einschrauben in den transgingivalen Implantatkopf und auf der gegenüberliegenden Seite einen Stift zur Befestigung an der Suprastruktur trägt, verwendet werden. Beim Auftreten einer Angulation wird eine Hülse aus ausbrennbarem Kunststoff entweder subtraktiv durch mechanischen Abtrag oder additiv durch Aufbringen von Wachs individuell geformt. Das so erzeugte Modell wird anschließend nach der Gussmethode des verlorenen Modells aus Metall gegossen, wobei eine Schraube in Längsrichtung durch die Hülse geführt wird und die Stifthülse am transgingivalen Implantatkopf mittels dieser Schraube verschraubt wird. Alternativ dazu kann der Aufbaustift auch komplett mittels CAD/CAM hergestellt werden.

Des Weiteren wird eine individuell an die Diagnosedaten des Patienten angepasste Suprastruktur, wie z. B. eine Krone oder Brücke, hergestellt. Dabei können für die Unterseite der Suprastruktur, die als Anschlussfläche an das Implantat dient, für die Formung des Randes die bereits vorhandenen geometrischen Daten der Präparationsgrenze und für die Formung der Topologie der Fläche der Unterseite die invertierten Daten der Fläche des Implantatkopfes verwendet werden.

Nach erfolgter Einheilung des Implantats wird der Aufbaustift in die auf dem Implantatkopf befindliche Gewindebohrung eingeschraubt.

Anschließend wird die entsprechend der Form des Implantatkopfes geformte Unterseite der Suprastruktur auf die gesamte Fläche des Implantatkopfes aufgeklebt bzw. aufzementiert. Im selben Schritt wird der Aufbaustift mit der Suprastruktur mechanisch verbunden.

Die Erfindung wird nachfolgend anhand von vier Ausführungsbeispielen näher erläutert; hierzu zeigen in schematischer Darstellung zunächst:
- Fig. 1:: ein Zahnimplantat mit Krone (Schnitt; Ansicht von vestibulär)
- Fig. 2:: ein Zahnimplantat (Schnitt; Ansicht von approximal)
- Fig. 3:: einen Implantatkopf in Draufsicht

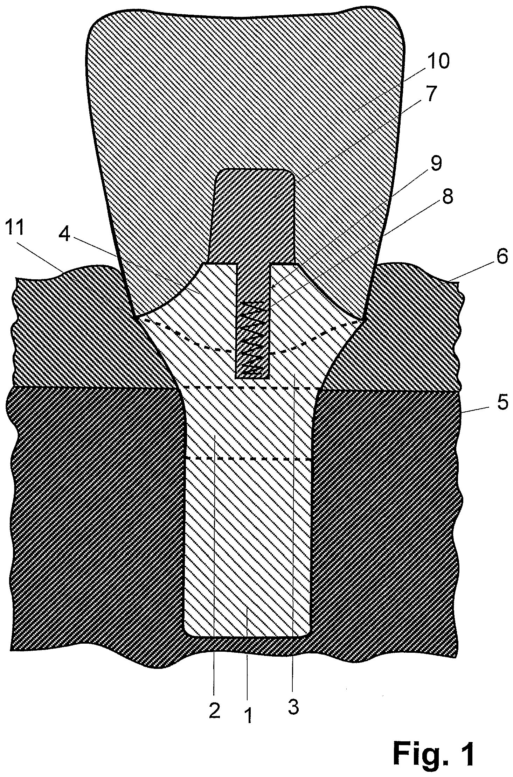

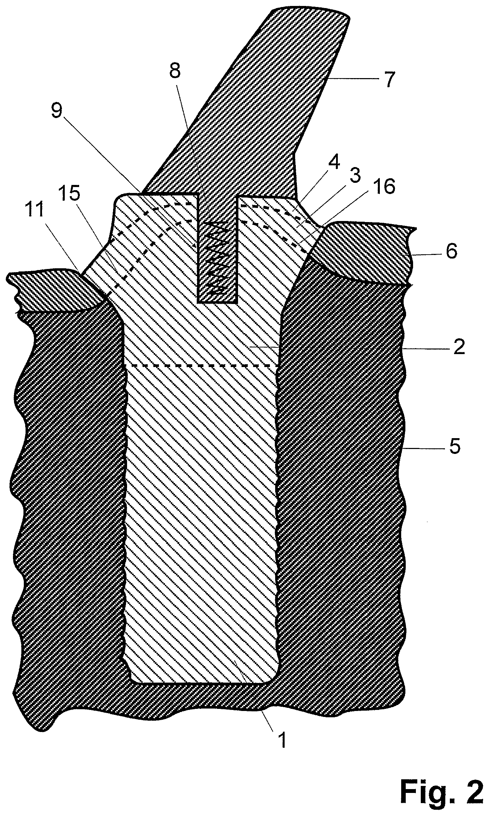

In Fig. 1 ist ein einteiliges Zahnimplantat aus Titan mit einer daran befestigten Krone 10 als Querschnitt mit palatinaler Blickrichtung dargestellt. Zur besseren Verdeutlichung zeigt Fig. 2 ein Implantat mit prothetischem Aufbaustift 7 im Querschnitt, jedoch mit einer Blickrichtung in Richtung des Kieferknochens.

Das Implantat besteht aus einem unteren enossalen Bereich 1, einem oberen enossalen Bereich 2, einem transgingivalen Bereich 3 und einem transgingivalen Implantatkopf 4. Zur Herstellung des Implantats werden Bereich 1 und Bereich 2 mit Hilfe von CAD/CAM sowie Bereich 3 und der transgingivale Implantatkopf 4 mit Hilfe von CAD/CAM oder durch manuelle Bearbeitung aus einem Rohling geformt.

Der tief im Kieferknochen sitzende, untere enossale Bereich 1 weist ein Wabenmuster auf, wobei sich jeweils in den Ecken der Waben Spitzen befinden. Diese bewährte Struktur ermöglicht ein optimales Einwachsen des Implantats.

An den unteren enossalen Bereich 1 schließt sich der obere enossale Bereich 2 an, der so ausgeformt ist, dass sein oberer Rand genau bis zur Oberkante des kammförmigen Kieferknochens 5 reicht. Um dies zu erreichen wird üblicherweise der obere enossale Bereich 2 auf der vestibulären Seite wesentlich kürzer und auf der palatinalen Seite etwas kürzer ausgeführt als an den restlichen Stellen. Es gibt jedoch auch Fälle, in denen zur genauen Anpassung an die Kammform des Kiefers die palatinale Seite kürzer ausgeführt werden muss als die vestibuläre. Knochendehiszenzen, die durch das Bohren des Implantatbettes entstehen, werden durch diese spezielle Formgebung vollständig anatomisch erfasst. Um außerdem eine breitere Auflagefläche und eine bessere Kräfteverteilung im Knochen 5 zu erreichen, wird der obere enossale Bereich 2 nach oben hin breiter (Trichterform). Aufgrund seiner aufgerauten Oberfläche kann der Bereich 2 auch mit dem Kieferknochen 5 verwachsen.

Dem Bereich 2 folgt der glatt polierte transgingivale Bereich 3. Dieser Bereich ist derart geformt, dass er umlaufend bis ca. 1 mm unterhalb des Zahnfleischsaums reicht, was in etwa der Tiefe entspricht, bis zu welcher auch bei einem gesunden Zahn das Zahnfleisch nicht mit dem Zahn verwachsen ist, sondern lediglich lose anliegt. Die glatt polierte Titanoberfläche ermöglicht hierbei ein gutes und reizfreies Anwachsen des Zahnfleisches 6.

An das Ende des transgingivalen Bereichs 3, welches gleichzeitig die Präparationsgrenze 11 bildet, schließt sich der transgingivale Implantatkopf 4 an. Der Implantatkopf 4 besteht aus einem flachen, nahezu ebenen inneren Teilbereich 12 und einen diesen umgebenden, steiler ausgeformten Teilbereich 13; die Bereiche 12/13 können (hier nicht dargestellt) auch übergangslos, etwa in balliger Form, zusammengefasst sein. Der steilere Teilbereich 13 verbindet den ebenen Teilbereich 12 mit dem transgingivalen Bereich 3. Bedingt durch die Anpassung des Implantats an die Kammform des Kieferknochens 5 ist der Teilbereich 13 in vestibulärer/bukkaler Richtung meistens wesentlich länger und steiler und in palatinaler/lingualer Richtung etwas länger und steiler als an den restlichen Stellen.

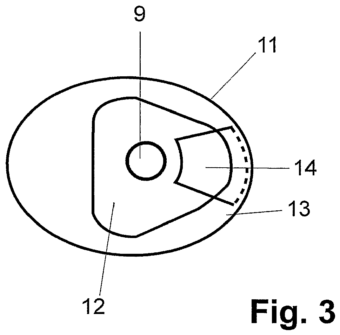

Wie aus Fig. 3 ersichtlich ist, befindet sich im flachen Teilbereich 12 des Implantats eine Gewindebohrung für die Befestigung des prothetischen Aufbaustifts. Außerdem ist im Teilbereich 12 eine Nut 14 in Form eines Kreissegmentstückes eingelassen, die ein Verdrehen der Krone verhindert; hierfür ist auf der Unterseite der Krone 10 ein entsprechendes Gegenstück ausgeformt. Wie in Fig. 2 zu sehen ist, wird ein individuell erstellter prothetischer Aufbaustift 7 verwendet. Dieser greift ebenfalls in die Nut 14 ein und ist somit auch gegen Verdrehen gesichert. Wird ein gerader, standardisierter Aufbaustift 7 verwendet, so greift dieser nicht in die Nut 14 ein, sondern es wird lediglich dessen stiftförmiger Teil angeschrägt. Beim später erfolgenden Einzementieren des Stiftes in die Krone 10, die ihrerseits über die Nut 14 und die eigentliche Form des Implantatkopfes 4 bis hin zur Präparationsgrenze 11 verdrehsicher am Implantat befestigt ist, wird dieser dann verdrehsicher mit der Krone 10 verbunden.

Die als Anschluss an das Implantat dienende Unterseite der Krone 10 ist genau an die Form des Implantatkopfes 4 bis hin zur Präparationsgrenze 11 angepasst. Dabei ist es von Vorteil, dass für die Gestaltung des Randes der Unterseite die bereits vorhandenen geometrischen Daten der Präparationsgrenze 11 und für die Herstellung der Fläche der Unterseite die invertierten geometrischen Daten des Implantatkopfes 4 verwendet werden können.

Die derart angepasste Unterseite der Krone 10 ist vollflächig und spaltfrei mit dem transgingivalen Implantatkopf 4 durch Zement bzw. Kleber verbunden. Eventuell beim Verkleben oder Zementieren austretendes Material kann aufgrund der guten Zugänglichkeit der Präparationsgrenze 11 unkompliziert entfernt werden. Zusätzlich ist in die Krone der glatte Teil des prothetischen Aufbaustifts 7 einzementiert. Der Aufbaustift 7 ist mit der anderen, schraubenförmigen Seite in den Implantatkopf 4 eingeschraubt. Hierbei nimmt die ganzflächige Verklebung zwischen Krone 10 und Implantat einen großen Anteil der beim Kauen auftretenden Kräfte auf. Die über den Prothetikposten 7 hergestellte Verbindung und flächige Auflage auf dem flachen Teil 12 des Implantatkopfes 4 dient dem Verhindern einer seitlichen Verschiebung und unterstützend der Aufnahme und Weiterleitung von Scherkräften.

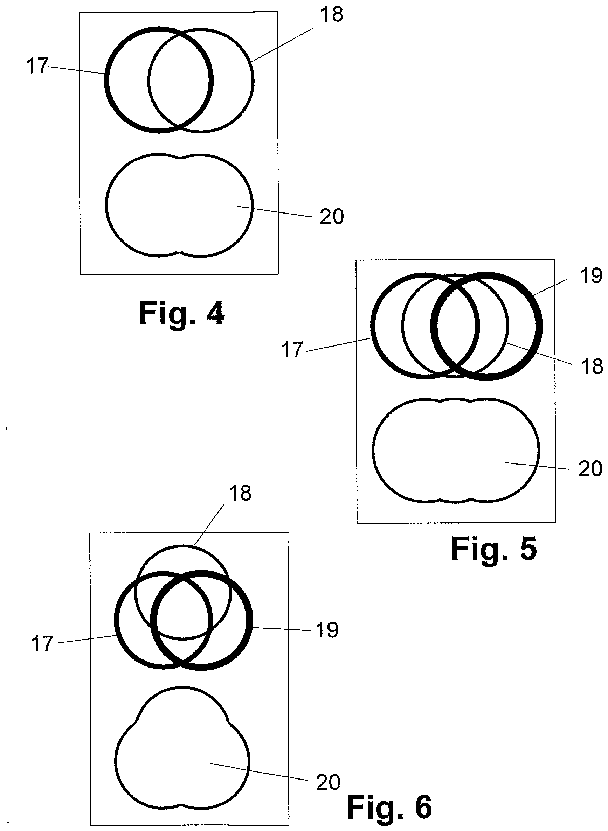

Fig. 4 bis 7 zeigen nämlich das Einbringen der Bohrungen in den Kieferkamm, die die Form des Implantatbettes erzeugen und letztlich die Geometrie des enossalen Bereichs des Zahnimplantatsystems bestimmen. Im Einzelnen sind dargestellt:
- Fig. 4:: das Einbringen zweier zueinander versetzter Bohrungen und das dadurch entstandene (8-förmige) Implantatbett in Draufsicht;
- Fig. 5:: das Einbringen dreier in Form einer Linie beabstandeter und gegeneinander verkippter (nicht ersichtlich) Bohrungen in Draufsicht;
- Fig. 6:: das Einbringen dreier in Form eines gleichseitigen Dreiecks beabstandeter und gegeneinander verkippter (nicht ersichtlich) Bohrungen in Draufsicht;

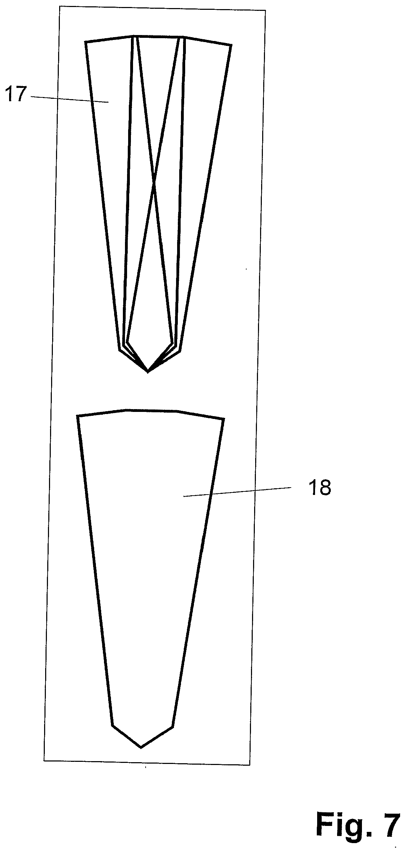

- Fig. 7:: das Einbringen dreier in einer Linie beabstandeter und gegeneinander verkippter Bohrungen in seitlicher Ansicht.

In den Figuren 4 bis 6 sind im oberen Teil die Positionen der einzelnen für das Implantatbett erforderlichen Bohrungen 17-19 und im unteren Teil die Form des Implantatbettes 20 dargestellt, das sich durch deren Überlagerung ergibt. Die Bohrungen sind zylindrisch und haben denselben Durchmesser.

In Fig. 4 sind zwei, in Fig. 5 drei in einer Linie und in Fig. 6 drei in Form eines gleichseitigen Dreiecks beabstandete und gegeneinander verkippte Bohrungen 17-19 dargestellt. Bei den in einer Linie angeordneten Bohrungen 17-19 und dem daraus geformten Implantatbett 20 hält das Implantat vergleichsweise höheren Drehbelastungen stand, während es bei einem Implantatbett, das in Form eines gleichseitigen Dreiecks angeordneten Bohrungen 17-19 gebildet ist, unempfindlicher gegenüber Kippmomenten ist.

Aus Fig. 7 ist ersichtlich, dass sich einerseits die Spitzen der Bohrungen 17-19 im untersten Punkt des Implantatbetts 20 treffen und andererseits die Bohrungen 17-19 sich über die gesamte Länge des Implantatbetts überschneiden. Damit wird eine konische Form des Implantatbettes 20 bzw. des unteren enossalen Bereichs erreicht, bei der jede Querschnittsfläche zusammenhängend ist.

### Liste der verwendeten Bezugszeichen:

- 1: unterer enossaler Bereich
- 2: oberer enossaler Bereich
- 3: transgingivaler Bereich
- 4: transgingivaler Implantatkopf
- 5: Kieferknochen
- 6: Gingiva
- 7: prothetischer Aufbaustift
- 8: Gewinde
- 9: Gewindebohrung
- 10: Krone
- 11: Präparationsgrenze/Kante
- 12: flacher Teilbereich
- 13: steilerer Teilbereich
- 14: Nut
- 15: vestibuläre/buccale Verkürzung
- 16: palatinale/linguale Verkürzung
- 17-19: Bohrung
- 20: Implantatbett

## Patentansprüche

1. Zahnimplantatsystem bestehend aus einem Implantat und einer darauf aufgebauten Suprakonstruktion, wobei sich das Implantat aus einem wabenförmig strukturierten unteren enossalen Bereich (1), einem sich nach oben hin aufweitenden oberen enossalen Bereich (2) mit aufgerauter Oberfläche, einem transgingivalen Bereich (3) mit glatter Wandfläche und einem transgingivalen Implantatkopf (4) zusammensetzt und alle Bereiche naht- und spaltlos aneinander anschließen,
- wobei der obere enossale Bereich (2) jeweils in vestibulärer/buccaler Richtung eine Verkürzung (15) und in palatinaler/lingualer Richtung eine Verkürzung (16) aufweist, sodass der obere enossale Bereich (2), an den Stellen, an denen er seitlich am Kieferkamm anliegt, höher ausgeführt ist,
- und der transgingivale Bereich (3) bei jedem Längsschnitt und jedem Querschnitt eine andere Form aufweist als bei jedem davor oder danach liegenden Längsschnitt bzw. Querschnitt,
und der transgingivale Bereich (3) nach oben durch einen dreidimensional in Höhe, Breite und Tiefe differenziert geformten transgingivalen Implantatkopf (4) begrenzt wird, wobei eine umlaufende Kante (11), durch das Aneinandergrenzen des Implantatkopfes (4) und des transgingivalen Bereiches (3) gebildet ist, sodass nach erfolgter Implantierung die umlaufende Kante (11) unterhalb des Zahnfleischsaums verlaufen wird,
- wobei der Implantatkopf (4) ungefähr in seiner Mitte einen flachen Teilbereich (12) aufweist, an den sich diesen umgebend ein steiler abfallender Teilbereich (13) anschließt, durch welchen der flache Teilbereich (12) und der transgingivale Bereich (3) miteinander verbunden sind,
- wobei der steilere Teilbereich (13) des Implantatkopfes (4) in vestibulärer/buccaler Richtung sowie in palatinaler/lingualer Richtung länger und steiler als in den übrigen Richtungen ist.

2. Zahnimplantatsystem nach Anspruch 1, **dadurch gekennzeichnet, dass** das Implantat, bestehend aus dem unteren enossalen Bereich (1), dem oberen enossalen Bereich (2), dem transgingivalen Bereich (3) und dem transgingivalen Implantatkopf (4), einteilig ausgeführt ist.

3. Zahnimplantatsystem nach Anspruch 1 und 2, **dadurch gekennzeichnet, dass** der untere (1) und der obere (2) enossale Bereich des Implantats eine nichtrotationssymmetrische Geometrie aufweisen, die der Überlagerung von mindestens zwei rotationssymmetrischen geometrischen Körpern entspricht, deren Symmetrieachsen parallel verlaufen oder zueinander verkippt sind, dass sie sich zumindest im unteren enossalen Bereich schneiden, wobei sich die geometrischen Körper zumindest im oberen enossalen Bereich überlappen.

4. Zahnimplantatsystem nach Anspruch 3 **dadurch gekennzeichnet, dass** die Geometrie des oberen und unteren enossalen Bereichs des Implantats der Überlagerung von geometrischen Körpern, die die Form eines Zylinders mit einer kegelförmigen Spitze haben, entspricht, wobei sich die Symmetrieachsen der Zylinder im unteren Ende des unteren enossalen Bereichs schneiden.

5. Zahnimplantatsystem nach Anspruch 3 **dadurch gekennzeichnet, dass** die Geometrie des oberen und unteren enossalen Bereichs des Implantats der Überlagerung von geometrischen Körpern, die die Form eines Zylinders mit einer kegelförmigen Spitze haben, entspricht, wobei die Symmetrieachsen der Zylinder parallel verlaufen und in Form eines n-Ecks beabstandet sind.

6. Zahnimplantatssystem nach den Ansprüchen 1 bis 5, **dadurch gekennzeichnet, dass** das Implantat aus Titan besteht.

7. Zahnimplantatssystem nach den Ansprüchen 1 bis 5, **dadurch gekennzeichnet, dass** das Implantat aus Zirkonoxid besteht.

8. Zahnimplantatsystem nach den Ansprüchen 1 bis 7, **dadurch gekennzeichnet, dass** der transgingivale Implantatkopf (4) eine Aussparung (14), welche als Sicherung gegen ein Verdrehen der Suprakonstruktion (10) dient, aufweist.

9. Zahnimplantatsystem nach Anspruch 8, **dadurch gekennzeichnet, dass** die Aussparung (14) die Form eines Kreissegments hat.

10. Zahnimplantatsystem nach den Ansprüchen 8 und 9, **dadurch gekennzeichnet, dass** ein prothetischer Aufbaustift (7) in die Aussparung (14) eingreift.

## Claims

1. A dental implant consisting of an implant and a superstructure assembled thereon, wherein the implant is composed of a lower endosseous region (1) structured in a honeycomb-like manner, an upper endosseous region (2) widening upwardly and having a roughened surface, a transgingival region (3) with smooth wall surface, and a transgingival implant head (4), and all regions are connected to one another seamlessly and without gaps,
- wherein the upper endosseous region (2) has a shortening (15) in the vestibular/buccal direction and has a shortening (16) in the palatal/lingual direction, such that the upper endosseous region (2) is taller at the points at which it bears laterally against the alveolar ridge,
- and the transgingival region (3) in any longitudinal section and any cross-section has a shape different than in any longitudinal or cross-section disposed therebefore or thereafter,
and the transgingival region (3) is delimited upwardly by a transgingival implant head (4) which is three-dimensionally shaped differently in height, width and depth, wherein a peripheral edge (11) is formed by the arrangement of the implant head (4) and the transgingival region (3) bordering on one another, such that, following successful implantation, the peripheral edge (11) will run below the gingival margin,
- wherein the implant head (4) has a flat region (12) approximately in its middle, which flat region is adjoined by a steeply sloping region (13) surrounding said flat region, by means of which sloping region the flat region (12) and the transgingival region (3) are connected to each other,
- wherein the steep region (13) of the implant head (4) is longer and steeper in the vestibular/buccal direction and in the palatal/lingual direction than in the other directions.

2. The dental implant system according to claim 1, **characterised in that** the implant, consisting of the lower endosseous region (1), the upper endosseous region (2), the transgingival region (3), and the transgingival implant head (4), is formed in one piece.

3. The dental implant system according to claim 1 and 2, **characterised in that** the lower (1) and the upper (2) endosseous region of the implant have a non-rotationally symmetrical geometry corresponding to the superimposition of at least two rotationally symmetrical geometric bodies of which the axes of symmetry run parallel to one another or are tilted relative to one another such that they intersect one another at least in the lower endosseous region, wherein the geometric bodies overlap at least in the upper endosseous region.

4. The dental implant system according to claim 3, **characterised in that** the geometry of the upper and lower endosseous region of the implant corresponds to the superimposition of geometric bodies which have the form of a cylinder having a conical tip, wherein the axes of symmetry of the cylinders intersect one another in the lower end of the lower endosseous region.

5. The dental implant system according to claim 3, **characterised in that** the geometry of the upper and lower endosseous region of the implant corresponds to the superimposition of geometric bodies which have the form of a cylinder having a conical tip, wherein the axes of symmetry of the cylinders run parallel to one another and are spaced apart in the form of a shape having n sides.

6. The dental implant system according to any one of claims 1 to 5, **characterised in that** the implant consists of titanium.

7. The dental implant system according to any one of claims 1 to 5, **characterised in that** the implant consists of zirconium oxide.

8. The dental implant system according to any one of claims 1 to 7, **characterised in that** the transgingival implant head (4) has a recess (14) which serves as a means for preventing rotation of the superstructure (10).

9. The dental implant system according to claim 8, **characterised in that** the recess (14) has the form of a segment of a circle.

10. The dental implant system according to claims 8 and 9, **characterised in that** a prosthetic assembly post (7) engages in the recess (14).

## Revendications

1. Implant dentaire constitué d'un implant et d'une supra-structure y étant montée, où l'implant est composé par une zone intra-osseuse inférieure (1) structurée sous forme d'alvéoles, d'une zone intra-osseuse supérieure (2) s'élargissant vers le haut avec une surface rendue rugueuse, une zone trans-gingivale (3) avec une surface de paroi lisse et une tête d'implant (4) trans-gingivale et toutes les zones sont jointives les unes aux autres sans couture et sans fente,
- où la zone intra-osseuse supérieure (2) présente respectivement, dans une direction vestibulaire/buccale, un raccourcissement (15) et dans la direction palatine/linguale, un raccourcissement (16), de sorte que la zone intra-osseuse supérieure (2) est conçue plus haute aux endroits sur lesquels la crête alvéolaire maxillaire est adjacente,
- et la zone trans-gingivale (3) présente une autre forme pour chaque coupe longitudinale et chaque coupe transversale, par rapport à chaque coupe longitudinale, respectivement coupe transversale, se situant avant ou après,
et la zone trans-gingivale (3) est délimitée vers le haut par une tête d'implant (4) trans-gingivale formée en trois dimensions avec une hauteur, une largeur et une profondeur différentes, où une arête périphérique (11) est formée par la frontière contigüe de la tête d'implant (4) et de la zone trans-gingivale (3), de sorte qu'après une implantation réussie, l'arête périphérique (11) s'étendra en dessous de la base des gencives,
- où la tête d'implant (4) présente une zone partielle plane (12) à peu près à son milieu, à laquelle est adjacente une zone partielle tombant de manière raide (13) sur la périphérie, par laquelle la zone partielle plane (12) et la zone trans-gingivale (3) sont reliées ensemble,
- où la zone plus raide (13) de la tête d'implant (4) est plus longue dans la direction vestibulaire/buccale ainsi que dans la direction palatine/linguale et est plus raide que dans les autres directions.

2. Système d'implant dentaire selon la revendication 1, **caractérisé en ce que** l'implant, constitué de la zone intra-osseuse inférieure (1), de la zone intra-osseuse supérieure (2), de la zone trans-gingivale (3) et de la tête d'implant (4) transgingivale, est conçu en une seule pièce.

3. Système d'implant dentaire selon les revendications 1 et 2, **caractérisé en ce que** les zones intra-osseuses inférieure (1) et supérieure (2) de l'implant présentent une géométrie non symétrique en rotation, qui correspond à la superposition d'au moins deux corps géométriques symétriques en rotation, dont les axes de symétrie s'étendent parallèlement ou sont basculés l'un par rapport à l'autre, de sorte qu'ils se croisent au moins dans la zone intra-osseuse inférieure, où les corps géométriques se superposent au moins dans la zone intra-osseuse supérieure.

4. Système d'implant dentaire selon la revendication 3, **caractérisé en ce que** la géométrie des zones intra-osseuses supérieure et inférieure de l'implant correspond à la superposition de corps géométriques qui ont la forme d'un cylindre avec une pointe en forme de cône, où les axes de symétrie des cylindres se croisent à l'extrémité inférieure de la zone intra-osseuse inférieure.

5. Système d'implant dentaire selon la revendication 3, **caractérisé en ce que** la géométrie des zones intra-osseuses supérieure et inférieure de l'implant correspond à la superposition de corps géométriques qui ont la forme d'un cylindre avec une pointe en forme de cône, où les axes de symétrie des cylindres s'étendent parallèlement et sont espacés sous la forme d'un polygone à n angles.

6. Système d'implant dentaire selon les revendications 1 à 5, **caractérisé en ce que** l'implant est constitué de titane.

7. Système d'implant dentaire selon les revendications 1 à 5, **caractérisé en ce que** l'implant est constitué d'oxyde de zirconium.

8. Système d'implant dentaire selon les revendications 1 à 7, **caractérisé en ce que** la tête d'implant (4) trans-gingivale présente une cavité (14), laquelle sert de sécurité contre une rotation de la superstructure (10).

9. Système d'implant dentaire selon la revendication 8, **caractérisé en ce que** la cavité (14) a la forme d'un segment circulaire.

10. Système d'implant dentaire selon les revendications 8 et 9, **caractérisé en ce qu'**un pivot de prothèse (7) est en prise dans la cavité (14).
